# NOUVEAU FASCICULE DE BREVET EUROPEEN

(11) **EP 3 083 970 B2**
(45) Date de publication et mention de la décision concernant l'opposition: **13.03.2024**
(45) Mention de la délivrance du brevet: 04.09.2019
(21) Numéro de dépôt: 14828237.9
(22) Date de dépôt: 17.12.2014
(51) Int. Cl.: C12N 7/00, C12N 15/86

(54) **PROCÉDÉ DE PURIFICATION DE VIRUS OU VECTEURS VIRAUX ENVELOPPES**
VERFAHREN ZUR REINIGUNG VON BEHÜLLTEN VIREN ODER VIRALEN VEKTOREN
METHOD FOR PURIFYING ENVELOPED VIRUSES OR VIRAL VECTORS

(30) Priorité: 17.12.2013 FR 1362835
(43) Date de publication de la demande: 26.10.2016
(73) Titulaire: Genethon, 91000 Evry (FR)
(72) Inventeur: BOUDEFFA, Driss, Montreal, Québec QC H1T 3H4 (CA); MERTEN, Otto-Wilhelm, 78121 Crespieres (FR); FENARD, David, 91540 Mennecy (FR)
(74) Mandataire: Cabinet Becker et Associés
(86) Numéro de dépôt international: PCT/FR2014/053406
(87) Numéro de publication internationale: WO 2015/092287

(56) Documents cités:
- WO-A1-2009/153563
- WO-A2-2006/052813
- WO-A2-2007/123961
- US-A- 5 661 022
- RODRIGUES ET AL: "Purification of retroviral vectors for clinical application: Biological implications and technological challenges", JOURNAL OF BIOTECHNOLOGY, ELSEVIER SCIENCE PUBLISHERS, AMSTERDAM, NL, vol. 127, no. 3, décembre 2006 (2006-12), pages 520-541, XP005787045, ISSN: 0168-1656, DOI: 10.1016/J.JBIOTEC.2006.07.028
- Anonymous: "Purification of influenza A/H1N1 using CaptoTM Core 700", Application note 29-003-34 AA, mars 2012 (2012-03), pages 1-8, XP055141530, Extrait de l'Internet: URL:http://wolfson.huji.ac.il/purification /PDF/HCIC/GE_CaptoCore700PurificInfluenzaA H1N1.pdf [extrait le 2014-09-19]
- DE LAS MERCEDES SEGURA M ET AL: "Downstream processing of oncoretroviral and lentiviral gene therapy vectors", BIOTECHNOLOGY ADVANCES, ELSEVIER PUBLISHING, BARKING, GB, vol. 24, no. 3, mai 2006 (2006-05), pages 321-337, XP028005965, ISSN: 0734-9750, DOI: 10.1016/J.BIOTECHADV.2005.12.001 [extrait le 2006-05-01]
- S HERZER ET AL: "Isoelectric titration curves of viral particles as an evaluation tool for ion exchange chromatography", LIFE SCI NEWS, vol. 13, 2003, pages 16-18, XP055144022,
- MCTAGGART SALLY ET AL: "Effects of culture parameters on the production of retroviral vectors by a human packaging cell line", BIOTECHNOLOGY PROGRESS, AMERICAN INSTITUTE OF CHEMICAL ENGINEERS, US, vol. 16, no. 5, septembre 2000 (2000-09), pages 859-865, XP002333787, ISSN: 8756-7938, DOI: 10.1021/BP000078J
- MORIZONO K ET AL: "Transient low pH treatment enhances infection of lentiviral vector pseudotypes with a targeting Sindbis envelope", VIROLOGY, ELSEVIER, AMSTERDAM, NL, vol. 355, no. 1, 10 novembre 2006 (2006-11-10), pages 71-81, XP024896557, ISSN: 0042-6822, DOI: 10.1016/J.VIROL.2006.07.015 [extrait le 2006-11-10]
- H P Lesch et al: "Production and purification of lentiviral vectors generated in 293T suspension cells with baculoviral vectors", Gene Therapy, Nature Publishing Group, London, GB, vol. 18, no. 6, 1 June 2011 (2011-06-01), pages 531-538, GB ISSN: 0969-7128, DOI: 10.1038/gt.2010.162
- YAMADA KAORU; MCCARTY DOUGLAS M; MADDEN VICTORIA J; WALSH CHRISTOPHER E: "LENTIVIRUS VECTOR PURIFICATION USING ANION EXCHANGE HPLC LEADS TO IMPROVED GENE TRANSFER.", Biotechniques, Informa Healthcare, US, vol. 34, no. 5, 1 May 2003 (2003-05-01), pages 1074-1080, US ISSN: 0736-6205
- Vladimir Slepushkin et al: "Large-scale Purification of a Lentiviral Vector by Size Exclusion Chromatography or Mustang Q Ion Exchange Capsule", BioProcessing Journal, vol. 2, no. 5, 1 September 2003 (2003-09-01), pages 89-95,

## Description

### ARRIERE PLAN TECHNOLOGIQUE

Les vecteurs lentiviraux dérivés du virus de l'immunodéficience humaine (VIH-1 notamment) font partie des vecteurs les plus utilisés pour la thérapie génique. Ces vecteurs sont en général pseudotypés avec des glycoprotéines provenant d'autres virus: virus leucémogène du gibbon (GALV en anglais pour gibbon ape leukemia virus; glycoprotéines GaLV-TR), virus de la stomatite vésiculaire (VSV-g), virus de la rougeole (ou MV pour measles virus en anglais). Des procédés de purification de lots cliniques de vecteurs lentiviraux pseudotypés avec la protéine VSV-G ont été décrits (Schweizer et Merten, 2010). Cependant les vecteurs viraux pseudotypés avec d'autres protéines d'enveloppe, et plus particulièrement avec des glycoprotéines dérivées de GaLV ou MV, sont relativement peu utilisés car aucun protocole de purification satisfaisant n'est disponible à l'heure actuelle. L'obstacle limitant majeur pour la purification de ce type de vecteurs pseudotypés est lié à l'instabilité et à la fragilité de certaines glycoprotéines membranaires. Pourtant, ces vecteurs sont particulièrement intéressants au regard de leur tropisme moins large que celui des vecteurs pseudotypés avec la protéine VSV-G. Par exemple, les vecteurs pseudotypés avec une glycoprotéine dérivée de GALV possèdent un tropisme plus restreint et visent plus particulièrement les cellules souches hématopoïétiques. La mise à disposition d'un procédé efficace de purification de vecteurs pseudotypés avec des glycoprotéines GALV représente donc un enjeu majeur dans le domaine de la thérapie génique.

Les inventeurs se sont donc proposés de développer un procédé de purification de rétrovirus et notamment de virus pseudotypés par la glycoprotéine d'enveloppe GaLV ou par d'autres protéines d'enveloppe, en vue de produire des préparations de virus à usage clinique.

### RESUME DE L'INVENTION

La présente invention découle de l'observation inattendue faite par les inventeurs de l'influence du pH des solutions utilisées au cours de la purification d'un virus enveloppé, et de l'influence positive de certains additifs sur le rendement de ladite purification.

Les inventeurs ont pu montrer que l'addition d'un polyol dans un ou plusieurs des tampons utilisés au cours d'une ou plusieurs étapes d'un procédé de purification d'un virus enveloppé permettait d'obtenir une augmentation substantielle du rendement de purification. En particulier, l'amélioration du rendement d'une purification comprenant une étape d'ultrafiltration/diafiltration suivie d'une chromatographie échangeuse d'anions est observée lorsque les tampons utilisés au cours de cette chromatographie comprennent un polyol.

### DESCRIPTION DETAILLEE DE L'INVENTION

### Production de virus et vecteurs enveloppés

La production de virus ou vecteurs enveloppés est bien connue dans l'état de la technique. L'homme du métier peut se référer à ses connaissances générales dans ce domaine, notamment représentées par Ansorge et al. 2010; Schweizer and Merten 2010; Rodrigues et al. 2011.

La cellule hôte peut être choisie parmi toute cellule permettant la production d'un virus enveloppé. Selon un mode particulier de réalisation, ladite cellule est choisie parmi une cellule humaine (HEK293, HEK293T, HEK293FT, Te671, HT1080, CEM), une cellule de muridé (NIH-3T3), une cellule de mustélidé (Mpf), une cellule de canidé (D17) (Miller and Chen 1996 ; Miller 2001; Merten 2004 ; Rodrigues et al. 2011 ; Stacey and Merten 2011).

Dans les procédés employant des cellules transfectées de manière transitoire, tout agent permettant la transfection de plasmides peut être utilisé. Il peut être notamment fait emploi de phosphate de calcium ou de polyéthylèneimine, bien que d'autres agents puissent être envisagés par l'homme du métier (Ansorge et al. 2010). Les conditions (notamment quantité de plasmide(s), ratio entre les plasmides, ratio entre le(s) plasmide(s) et l'agent de transfection, le type de milieu, etc.) et la durée de transfection pourront être adaptées par l'homme du métier en fonction des caractéristiques du virus produit et/ou du transgène introduit dans le plasmide de transfert.

Selon un mode particulier de réalisation, le milieu de culture utilisé présente un pH neutre (e.g. compris entre 7 et 7,4, notamment 7, 7,1, 7,2, 7,3 ou 7,4) classiquement utilisé dans l'état de la technique pour la culture des cellules et la production de virus. Selon un mode particulier de réalisation, le procédé de production utilisé comprend la culture des cellules productrices en milieu modérément acide. Alors que l'état de la technique présente la neutralité des milieux de culture comme une condition nécessaire pour la culture optimale des cellules et la production optimale des virus et vecteurs enveloppés, il a été découvert que des conditions modérément acides permettaient au contraire d'améliorer de manière significative la production d'un virus enveloppé, notamment d'un lentivirus, en particulier d'un lentivirus pseudotypé (par exemple avec la protéine GaLV (ou GaLVTR), VSV-G, ou protéines d'enveloppe du virus de la rougeole).

### Purification de virus et vecteurs enveloppés

Le(s) tampon(s) utilisés au cours de l'étape d'ultrafiltration/diafiltration, plus particulièrement de TFF, peuvent également être des tampons acides, neutres ou basiques.

La solution chargée sur ou dans le dispositif d'ultrafiltration/diafiltration ou sur la colonne de chromatographie échangeuse d'anions peut correspondre au surnageant de culture cellulaire éventuellement prétraité au moyen d'une benzonase et/ou d'une centrifugation basse vitesse et/ou d'une clarification. Il est entendu que ce surnageant de culture éventuellement prétraité ne correspond pas à un "tampon de purification". Cependant, son pH peut également être ajusté avant chargement si besoin. Si la production a été réalisée à pH neutre ou acide, le surnageant de culture éventuellement prétraité peut être chargé directement, ou son pH peut être diminué ou augmenté avant chargement. Il est également possible d'envisager l'ajout d'additifs dans le surnageant de culture éventuellement prétraité avant chargement. Par exemple, il est possible d'ajouter dès cette étape un polyol, un antioxydant (notamment L-histidine, L-méthionine, L-cystéine, glutathion ou vitamine C), un sel métallique, notamment un sel de magnésium tel que le MgCl₂ ou MgSO₄, ou tout autre additif adéquat.

Selon un mode particulier de réalisation, le surnageant de culture, éventuellement prétraité, est chargé directement sur ou dans le dispositif d'ultrafiltration/diafiltration ou sur la colonne de chromatographie, sans ajustement de pH et sans ajout d'additif. Des tampons de pH acide, basique ou neutre peuvent être, et de préférence sont, utilisés au préalable pour équilibrer le dispositif d'ultrafiltration/diafiltration et/ou pour réaliser l'ultrafiltration/diafiltration ou la chromatographie échangeuse d'anions en tant que telle.

Selon une variante, l'étape d'ultrafiltration/diafiltration, est une étape de TFF. Dans cette variante, la diafiltration est réalisée avec un tampon dont le pH est ajusté selon les modalités exposées ci-dessus. Ainsi, le tampon utilisé peut être un tampon acide, notamment un tampon de pH compris entre 5 et 5,9 (notamment égal à 5,0, 5,1, 5,2, 5,3, 5,4, 5,5, 5,6, 5,7, 5,8 ou 5,9), en particulier un tampon de pH 5,5 à pH 5,9 (notamment égal à 5,5, 5,6, 5,7, 5,8 ou 5,9), plus particulièrement un tampon de pH 5,5. Dans une variante, le tampon utilisé est un tampon de pH compris entre 6 et 8 (notamment de pH égal à 6,0, 6,1 6,2, 6,3, 6,4, 6,5, 6,6, 6,7, 6,8, 6,9, 7,0, 7,1, 7,2, 7,3, 7,4, 7,5, 7,6, 7,7, 7,8, 7,9 ou 8,0), plus particulièrement entre 7 et 8 (notamment de pH égal à 7,0, 7,1, 7,2, 7,3, 7,4, 7,5, 7,6, 7,7, 7,8, 7,9 ou 8,0), comprenant un polyol.

Les tampons utilisés au cours de l'étape d'ultrafiltration et au cours de l'étape de diafiltration peuvent être différents ou identiques. Dans un mode particulier de réalisation, l'étape d'ultrafiltration est réalisée au moyen d'un tampon ayant un pH d'environ 7 (notamment un pH compris entre 6,8 et 7,2 (par exemple égal à 6,8, 6,9, 7,0, 7,1 ou 7,2), plus particulièrement un tampon de pH 7) et la diafiltration est réalisée au moyen d'un tampon ayant un pH compris entre 5 et 5,9 (notamment égal à 5,0, 5,1, 5,2, 5,3, 5,4, 5,5, 5,6, 5,7, 5,8 ou 5,9), plus particulièrement un tampon de pH compris entre 5,5 et 5,9 (notamment égal à 5,5, 5,6, 5,7, 5,8 ou 5,9), en particulier un tampon de pH égal à 5,5.

Dans un mode de réalisation, le procédé de purification comprend une étape de chromatographie échangeuse d'anions suivie d'une étape d'ultrafiltration/diafiltration. Dans un autre mode de réalisation, le procédé de purification comprend une étape d'ultrafiltration suivie d'une étape de chromatographie échangeuse d'anions.

Dans un mode particulier de réalisation, le procédé de purification comprend:
(a) la clarification du milieu de culture cellulaire;
(b) une étape d'ultrafiltration/diafiltration;
(c) une chromatographie échangeuse d'anions;
(d) une chromatographie d'exclusion;
les étapes (b) et (c) pouvant être inversées. Selon un mode préféré de réalisation, l'étape (c) suit l'étape (b).

Pour l'évaluation des fractions et la sélection de celles qui seront soumises à la suite du procédé de purification, la colonne peut être équipée en sortie d'un chromatographe équipé d'un lecteur d'absorbance UV 280 nm, d'un conductivimètre, d'une table traçante et d'un collecteur de fraction.

Dans le cadre de la présente invention, le terme "polyol" définit une molécule carbonée linéaire, cyclique ou bicyclique comprenant entre 3 et 18 atomes de carbone, en particulier entre 3 et 12 atomes de carbone, substituée par au moins 3-6 groupements hydroxyle, en particulier 8 groupement hydroxyle. Le polyol peut par exemple être un monosaccharide aldose ou kétose, notamment un tétrose, un pentose ou un hexose. On peut notamment citer les polyols monosaccharides suivants: érythrose, thréose, ribose, arabinose, xylose, lyxose, allose, altrose, glucose, mannose, gulose, idose, galactose, talose, érythrulose, ribulose, xylulose, fructose, psicose, sorbose, tagatose. Le polyol peut également être choisi parmi les disaccharides ou trisaccharides suivants qui constituent une liste non limitative d'autres polyols utilisables dans la mise en oeuvre de l'invention: cellobiose, gentiobiose, inulobiose, isomaltose, isomaltulose, kojibiose, lactose, lactulose, laminaribiose, leucrose, maltose, maltulose, mélibiose, nigerose, robinose, rutinose, sucrose, sophorose, tréhalose, tréhalulose, turanose, erlose, fucosyllactose, gentianose, inulotriose, 1-kestose, 6-kestose, maltotriose, mannotriose, mélézitose, néokestose, panose, raffinose, rhamninose. Dans un mode particulier de réalisation, le polyol est choisi parmi le raffinose, l'isomaltotriose, le sucrose, le mannitol, le sorbitol, le tréhalose, le glucose et le glycérol. Dans un mode particulier de réalisation, le polyol est le sucrose.

La concentration en polyol peut varier dans une large mesure, et peut en particulier être différente pour chacun des différents tampons mis en oeuvre au cours de cette étape. La concentration en polyol peut notamment être comprise entre 1% et 15% (p/v) notamment entre 1,5% et 10%, en particulier entre 2% et 8%, plus particulièrement entre 2% et 5%. Dans un mode particulier de réalisation, la concentration en polyol d'un ou plusieurs des tampons de la chromatographie échangeuse d'anions est de 5% (p/v). Dans un mode particulier de réalisation, tous les tampons utilisés au cours du procédé de purification comprennent un polyol, notamment du sucrose, en particulier à 5% (p/v). Ainsi, selon ce mode de réalisation le procédé peut comprendre une étape de TFF, une étape de chromatographie échangeuse d'anions et une étape de chromatographie d'exclusion au cours desquelles tous les tampons utilisés comprennent un polyol. Dans un autre mode de réalisation, les tampons des étapes de TFF et de chromatographie échangeuse d'anions comprennent un polyol alors que les tampons utilisés pour équilibrer et éluer la colonne de chromatographie d'exclusion ne comprennent pas de polyol, ces tampons correspondant au tampon de formulation dont la composition dépendra en grande partie de la destination thérapeutique et du mode d'administration du produit fini.

Dans un autre mode de réalisation, un ou plusieurs des tampons utilisés au cours du procédé de la présente invention, notamment les tampons utilisés au cours de l'ultrafiltration/diafiltration et/ou de la chromatographie échangeuse d'anions, comprennent un sel de magnésium, notamment du chlorure de magnésium ou du sulfate de magnésium. La concentration en sel de magnésium, en particulier en chlorure de magnésium ou en sulfate de magnésium dans chacun des tampons peut, indépendamment pour chaque tampon, être comprise entre 0,1 mM et 5 mM, notamment entre 1 et 3 mM, en particulier 2 mM.

Dans un autre mode de réalisation, un ou plusieurs des tampons utilisés au cours du procédé de la présente invention comprennent du L-His, du L-Met, du L-Cys, du glutathion, ou de vitamine C en vue d'inactiver des radicaux libres. La concentration en ces composants dans chacun des tampons peut, indépendamment pour chaque tampon, être comprise entre 0,1 mM et 20 mM.

Le procédé de purification selon l'invention peut également comprendre une ou plusieurs étapes de traitement du ou des échantillons avec une nucléase, notamment une benzonase. La nucléase peut être utilisée avant ou après chacune des étapes. Dans un mode de réalisation, la nucléase, en particulier la benzonase, est utilisée dans le milieu de culture des cellules productrices après l'étape de transfection des plasmides.

Selon un mode de réalisation, une ou plusieurs étape(s) de la purification sont réalisées à une température inférieure à la température ambiante, en particulier à une température comprise entre 2 et 12°C, plus particulièrement entre 4 et 10°C. Selon un mode particulier de réalisation, une, plusieurs ou toutes les étapes de la purification sont réalisées à environ 4°C.

### LEGENDE DES FIGURES

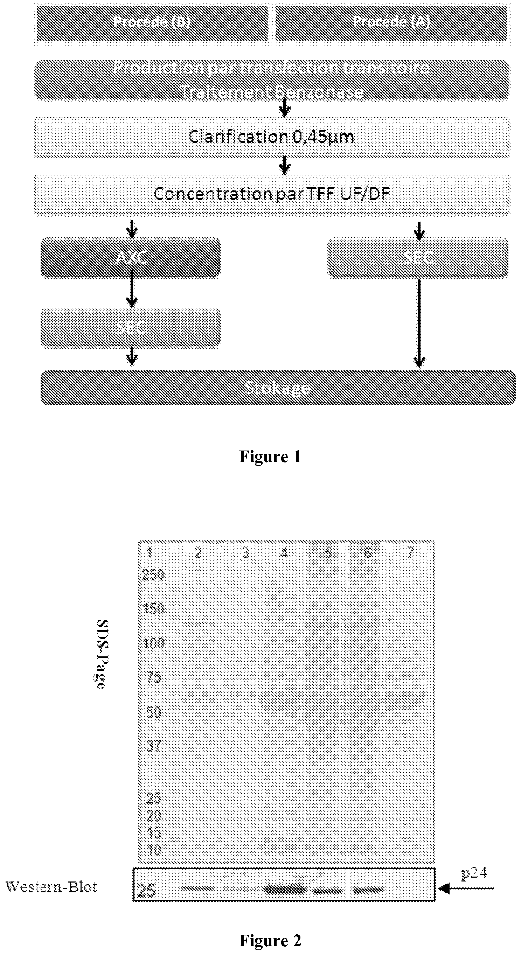

**Fig. 1****.** Deux procédés pour la purification des particules lentivirales LV-GaLV-TR : le procédé (A) est un procédé simplifié mettant en oeuvre une seule étape de chromatographie d'exclusion (filtration sur gel) après l'étape de la filtration tangentielle en flux; et le procédé (B) est un procédé plus élaboré en vue d'obtenir une pureté plus élevée que lors de l'utilisation du procédé (A) - par exemple en vue de la production des vecteurs pour utilisation clinique.
**Fig. 2****.** Comparaison des membranes avec une coupure de 500 kDa et de 750 kDa dans l'élimination des protéines contaminantes (SDS-PAGE (en haut) et Western Blot anti-p24 (en bas)). La bande à 24/25 kDa (= p24) est bien visible pour tous les échantillons sur le Western Blot : 1) marqueurs de taille ; 2) diafiltration (a) (essai 1) à 750 kDa ; 3) diafiltration (b) (essai 2) à 750 kDa ; 4) ultracentrifugation à 68338g pendant 3h (resuspension en milieu de culture X-vivo 20) ; 5) diafiltration (a) (essai 1) à 500 kDa ; 6) diafiltration (b) (essai 2) à 500 kDa ; 7) surnageant de culture contenant des vecteurs LV-GaLV-TR.

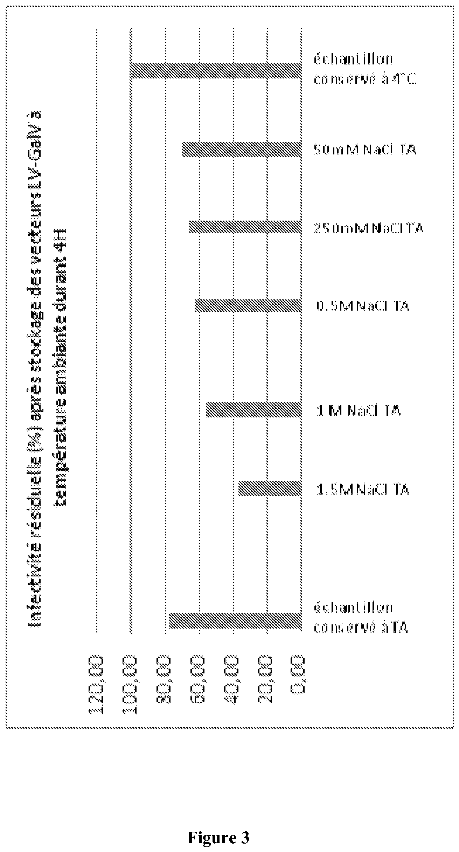

**Fig. 3****.** Effet du NaCl sur la stabilité des vecteurs LV-GaLV-TR codant une GFP stockés à température ambiante. Les vecteurs sont incubés pendant 4 heures à pH 7.0 (PBS) à température ambiante (TA). Pour optimiser l'étape de chromatographie échangeuse d'anions nous avons testé la stabilité des vecteurs dans un milieu salin NaCl. Pour cela les vecteurs ont été incubés après l'étape d'UF/DF dans des tampons PBS pH 7.0 de concentrations en NaCl différentes pendant 4h à température ambiante. Ensuite les vecteurs ont été titrés sur des cellules HCT116. 48 heures plus tard, les cellules sont passées au FACS pour mesurer le pourcentage d'expression de la GFP.

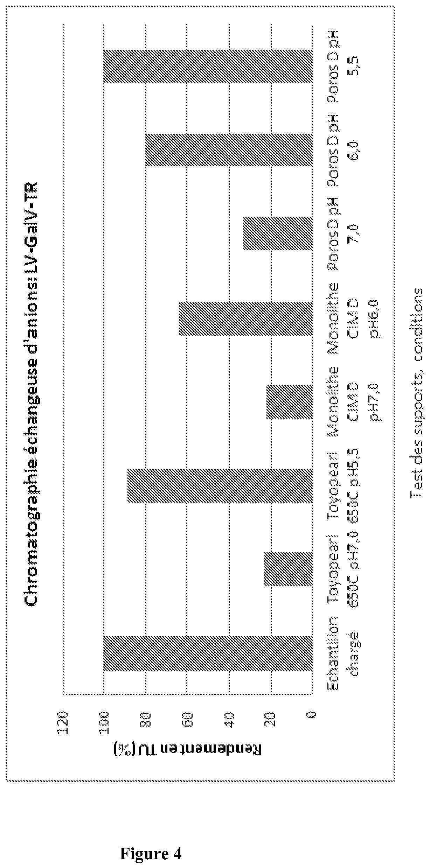

**Fig. 4****.** Effet du pH et de la salinité du tampon d'élution sur le rendement de purification des vecteurs lentiviraux infectieux après une étape de chromatographie échangeuse d'anions. La préparation de vecteurs a été produite par transfection de cellules HEK293T, clarifiée et concentrée/diafiltrée par TFF en vue d'être utilisée pour l'évaluation de différents supports de chromatographie échangeuse d'anions (faible). Différents supports ont été évalués : Toyopearl 650C DEAE, CIM D (DEAE) et Poros D. Le rendement 100% est équivalent au titre infectieux après l'étape précédente de TFF.

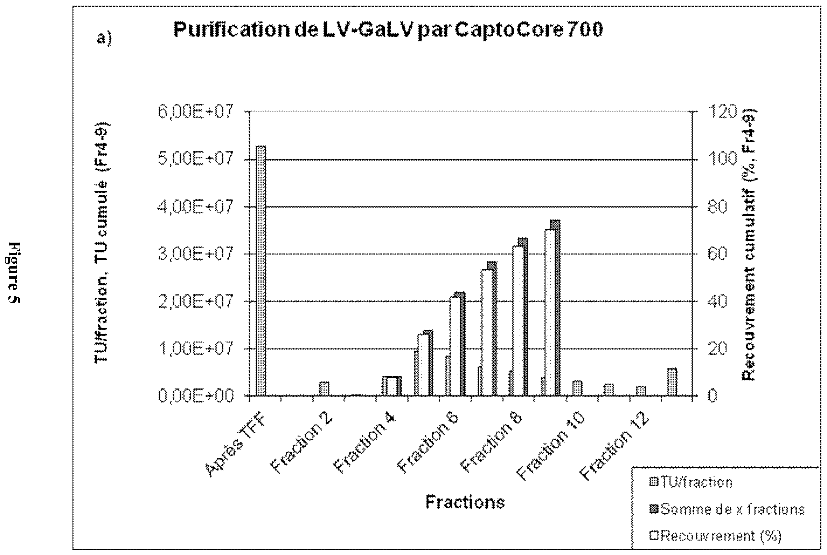

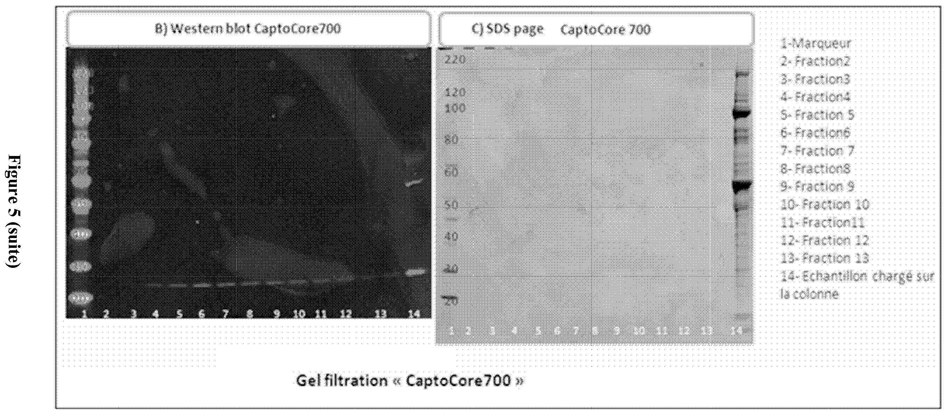

**Fig. 5****.** Purification d'une préparation de vecteurs lentiviraux GaLV-TR par chromatographie d'exclusion (Capto Core 700). Trois ml d'une préparation lentivirale a été concentrée/diafiltrée et ensuite passée sur une colonne de Capto Core 700 (4.5ml). Un tampon PBS (pH 7.0), sucrose 5%, MgCl₂ 2mM a été utilisé au cours de cette étape pour l'équilibrage de la colonne et la formulation. Des fractions de 1ml ont été récoltées et analysées pour la concentration en vecteur (TU) : a) Chromatogramme montrant le titre (TU) par fraction, le cumul de la quantité de vecteur pour les fractions 4-9 et le recouvrement (%) cumulatif pour les fractions 4-9 ; b) Western blot de toutes les fractions ; c) SDS-PAGE de toutes les fractions.

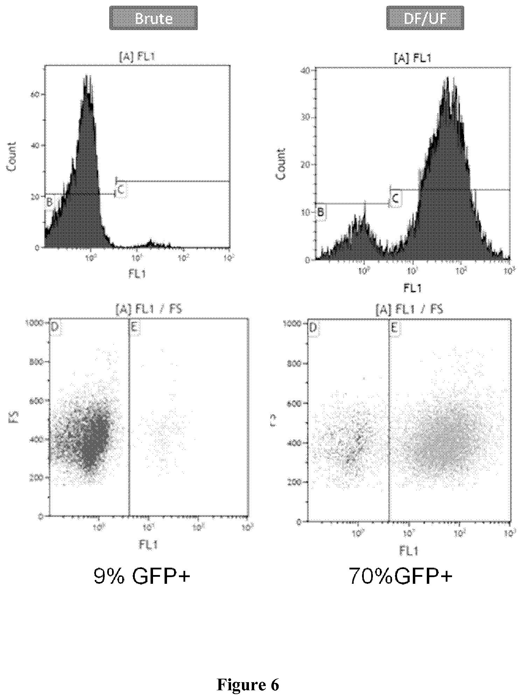

**Fig. 6****.** Transduction des cellules CD34+SC (sang du cordon ambilicale) avec un vecteur HIV-GaLV-TR de MOI 20 Brut: pourcentage de cellules exprimant la GFP déterminé par cytométrie en flux des cellules CD34+ transduites avec le produit brut HIV-GaLV-TR,
   DF/UF: pourcentage de cellules exprimant la GFP déterminé par cytométrie en flux des cellules CD34+ transduites avec la préparation de vecteurs HIV-GaLV-TR obtenus après purification et concentration par TFF du produit brut.

### EXEMPLES

Les travaux rapportés dans la présente demande ont bénéficié d'un financement par l'intermédiaire du 7^{ème} Programme Cadre de la Communauté Européenne (FP7/2007-2013), sous le numéro 222878.

### Matériel et méthodes

**Cellules:** les lignées cellulaires HEK293T et HCT116 (cellules cancéreuses colorectales CCL-247, origine : ATCC) sont cultivées à 37°C, 5% CO₂ dans du Dulbecco's modified Eagle's medium (Gibco) (DMEM+Glutamax) supplémenté avec 2 à 10% de sérum de veau foetal (SVF) (Life Technologies). Milieu de culture : DMEM/SVF tamponné à pH 6.0 par l'ajout d'acide chlorhydrique (HCl 37%, Sigma-Aldrich), puis filtré au moyen d'un filtre Corning^{®} 1000mL (0.22µm PES (polyethersulfone)).

### Production des vecteurs viraux:

Les vecteurs viraux dérivés de HIV-1 pseudotypés avec différentes glycoprotéines sont produits par quadri-transfection transitoire au phosphate de calcium dans des cellules 293T, par 4 plasmides comme décrit par Merten et al. (2011). 2x10⁸ cellules 293T sont ensemencées dans un Hyperflask 1760-cm² (Corning) dans 550ml de DMEM 10% SVF (Kutner et al. 2009). 24 heures plus tard, le milieu de culture est remplacé par le milieu de transfection, en combinant dedans le complexe ADN/CaCl₂/HBS. Les 4 plasmides : gagpol (pKLgagpol) 136µg, rev (pKrev) 52.25µg, plasmide transgène (pCCL-eGFP) 206.8µg, plasmide d'enveloppe approprié à chaque pseudotype : GaLV-TR : pBA.GALV-TR/Ampho-Kana (Gibbon ape Leukemia Virus) 223µg pour générer des LV-GaLV-TR; VSV-GpMDG (Vesicular stomatitis virus-g) 68.13µg pour générer des LV-VSV-g ; pFΔ30 et pHCMH2 (protéines d'enveloppe modifiées du virus de la rougeole) 40µg et 14µg pour générer les LV-MV ; quantité suffisante pour 18ml H₂O et 8.9ml TE0.1X, mélanger avec 3ml de CaCl₂ (2.5M) ensuite rajouter 30ml de HBS2X, attendre la formation du complexe pendant 4 min et ajouter le mix au milieu de culture. Après 16 heures le surnageant est remplacé par le milieu frais 2% SVF 15U Benzonase (Merck) et 2mM MgCl₂ (Sigma-Aldrich). La récolte se fait après 48 heures post transfection le surnageant est filtré sur filtre 0.45µm en acétate de cellulose (CA) 1L (Corning).

Les vecteurs rétroviraux MLV-GaLV sont produits par les cellules PG13. Ce sont des cellules productrices des vecteurs MLV-GaLV (Miller et al. 1991), les cellules sont maintenues dans du Dulbecco's modified Eagle's medium (Gibco) (DMEM+Glutamax) supplémenté avec 2 à 10% de SVF, à 37°C, 5% CO₂. La récolte des vecteurs se fait après 24 heures de changement de milieu de culture. Ensuite le surnageant de production est clarifié sur un filtre de 0.45µm en acétate de cellulose (Corning).

### Concentration des vecteurs viraux par filtration à flux tangentiel (TFF):

Cette étape consiste à concentrer le surnageant de production puis à remplacer le milieu de culture par un tampon adéquat pour la suite du procédé.

L'ultrafiltration (UF) est réalisée après la préparation de la cassette d'UF et la détermination de la perméabilité normalisée à l'eau NWP à 0,5 bar à 20°C. La membrane est ensuite équilibrée avec un tampon Bis-Tris pH 6,0 5% sucrose, 2mM MgCl₂ ou avec d'autres tampons en vue d'effectuer cette concentration/diafiltration à d'autres pHs (par ex. : PBS, pH 7,0, 5%M , sucrose, 2mM MgCl₂). Tout le procédé est réalisé à environ 4°C et le réservoir du produit à concentrer mis dans un bac à glace.

Principe : Une première concentration jusqu'au volume de 20 ml suivie d'une diafiltration avec 10 volumes de tampon de chargement de chromatographie échangeuse d'ions (dans ce cas : 10 x 20ml) sont réalisées. Ces étapes sont suivies d'une seconde concentration jusqu'au volume minimal possible (10 ml dans le cas présent).

Membrane 750 kDa, 410 cm² : « Hollow fiber cartridge » (GE Healthcare, Ref: UFP750-E3MA) en utilisant le Kros-Flow research II TFF system (Spectrum).

Après la validation de l'intégrité de la membrane, la concentration des vecteurs commence avec un volume de départ de 500ml de surnageant brut et est concentré par la membrane de 500ml à 20ml.

Le produit concentré est diafiltré contre 200ml de Tampon A (en vue de diafiltrer 10 fois 20ml de concentré). Cela représente un facteur de concentration de 25X. Le volume final du diafiltrat est de 10ml. Dans ce cas le facteur de concentration est de 50X.

### Chromatographie échangeuse d'anions :

Dans un premier protocole, l'étape de chromatographie échangeuse d'anions est réalisée en aval de la TFF. Plusieurs supports de chromatographie sont testés: colonne monolithe CIMD DEAE, CIMD Q (BIAseparation, Villach, Autriche), volume de la colonne : 1ml ; Sartobind D 75MA, volume : 2.1ml (Sartorius Stedim Biotech) ; Poros PI, volume de la colonne: 4ml ; Poros D 50, volume de la colonne: 4ml, Poros HQ, volume de la colonne: 4ml (LifeTechnologies), Toyopearl 650C DEAE, volume de la colonne: 2ml (Tosoh).

La colonne à tester est connectée à un chromatographe Biologic-LP (Biorad) équipé d'un lecteur d'absorbance UV 280, d'un conductivimètre, une table traçante (Chart recorder 1327, Bio-Rad), et d'un collecteur de Fraction (Model 2110, Bio-Rad).

La colonne est équilibrée avec 5 volumes de colonne (5 CV) de tampon A à 2ml/min. Après chargement de l'échantillon sur la colonne, la colonne est lavée avec 5 CV de tampon d'équilibrage approprié selon le pH souhaité, selon le tableau A). Une élution en deux étapes est ensuite réalisée : 0,3M NaCl, 20mM Bis-Tris, 5% sucrose, 2mM MgCl₂ (pH 6,0) puis 650mM NaCl, 20mM Bis-Tris, 5% sucrose, 2mM MgCl₂ (pH 6,0) pour éluer les vecteurs. Trois autres pH sont testés pH 5,5, 7,0 et 8,0 en utilisant les tampons appropriés (incluant, les tampons tels que Bis-propane, PBS, L-His); en présence (5%) et en absence de sucrose 5% et de MgCl₂ (2 mM).

Enfin la fraction est chargée tout de suite sur la colonne de filtration sur gel (chromatographie d'exclusion) pour éliminer les sels et protéines contaminants élués avec les vecteurs à 650mM NaCl.

Dans un second protocole, le surnageant de production clarifié est chargé sur une colonne de chromatographie échangeuse d'anion Poros D, sans étape préalable d'ultrafiltration/diafiltration pour évaluer le rendement de la chromatographie dans ces conditions. Le tampon d'équilibrage utilisé présente un pH de 5,5, et contient 5 % de sucrose et 2 mM de MgCl₂.

### Chromatographie d'exclusion :

C'est l'étape finale avant la filtration stérilisante pour les procédés A et B (figure 1). Cette étape consiste à éliminer les contaminants ayant une taille inferieure à celle du gel utilisé (exp: 750kDa, ou bien 500kDa). La colonne Captocore700 a été utilisée pour cette étape. Il s'agit d'un gel à double fonctionnalité: chromatographie d'exclusion et gel de chromatographie d'adsorption.

Avant de commencer le chargement, la colonne est sanitisée avec 1M NaOH et équilibrée avec le tampon de formulation. Le produit UF/DF (procédé A), ou les fractions correspondant au pic de chromatographie de chromatographie échangeuse d'anions (AXC) (procédé B) est chargé sur la colonne. 8 ml (UF/DF, ou fraction AXC) sont chargés à un débit de 0.5ml/min. Ensuite le tampon de formulation est injecté à 0.5ml/min (5 CV de tampon de formulation). La fraction correspond au pic UV est collectée (environ 16 ml) puis filtrée sur filtre 0.22µm (filtration stérilisante). Les échantillons sont stockés à -80°C.

### Titration des vecteurs viraux :

Le titre viral en unités de transduction (TU) des vecteurs ayant le gène rapporteur eGFP, est analysé par transduction des cellules HCT116. 72 heures post transduction les cellules sont passées au FACS pour déterminer le titre en TU/ml comme décrit précédemment (Pfeifer et al. 2009). Pour l'analyse physique des particules virales, le KIT ELISA p24 (PerkinElmer) a été utilisé pour la quantification de la protéine de capside des lentivirus p24 selon les instructions du fournisseur.

### Transduction des cellules de sang du cordon ombilical CD34+ :

Les cellules CD34 + sont isolées de sang du cordon ombilical, par sélection immunomagnétique (Miltenyi Biotec). La culture et la transduction des cellules CD34+ se fait comme décrit (Charrier et al. 2011) : premièrement les cellules sont préstimulées pendant la nuit dans un milieu X-Vivo 20 (Lonza) et supplémentées avec des cytokines. Les cellules pré-activées sont ensemencées dans une plaque 48 puits (5^{e}4 cellules/100µl). La transduction se fait par l'ajout de 100µl de vecteurs (1^{e}6TU) purifié en présence de 8µg/ml de vectofusine-1 (Fenard et al., 2013). Après 6 heures d'incubation on ajoute 1ml de milieu de différenciation (X-VIVO-20 complémenté avec 10% sérum, et en présence des Cytokines (hSCF, h-Il-3 h-Flt3 h-Il-6) comme décrit (Charrier et al. 2011)) dans chaque puits, et après 5 jours l'efficacité de transduction est évaluée par mesure de l'expression de la GFP par FACS (FC500, BD Biosciences).

### SDS-page Western Blot :

Les échantillons de culture contenant les vecteurs lentiviraux ou les échantillons purifiés, sont analysés par SDS-PAGE et par Western Blot afin de détecter la présence des protéines de capside p24. La révélation des protéines p24 est effectuée suivant la méthode développée par LI-COR, avec l'appareil Odyssey et le logiciel Odyssey 2.1. L'anticorps primaire utilisé est un anti-p24 (Santa-Cruz # SC-57823) pour la détection des protéines de capside p24 du VIH. L'anticorps est utilisé avec une dilution de 1/200ème en PBS1X-Tween 0.1% + blocker Odyssey (1:1). L'anticorps secondaire de chèvre utilisé couplé au fluorochrome « Dey 800 » de Li-COR est dirigé contre les anticorps primaires.

### Quantification des protéines résiduelles et de l'ADN résiduel spécifique :

Les protéines totales sont quantifiées par méthode de Bradford (Bio-Rad) avec l'albumine sérique comme standard. Le test se fait selon les instructions du fournisseur.

ADN résiduel : la quantification de l'ADN résiduel d'origine plasmidique et/ou provenant de la cellule hôte, se fait par PCR quantitative. Les échantillons sont traités à la protéinase K (Roche) puis l'ADN est extrait en utilisant le système MagNA Pure ADN and viral NA small volume kit (MagNA Pure 96 Roche). Une PCR quantitative en temps réel est ensuite effectuée, avec des amorces spécifiques pour le gène de la kanamycine afin de détecter l'ADN résiduel d'origine plasmidique. Pour détecter l'ADN résiduel de la cellule hôte on utilise des amorces qui ciblent le gène E1A. La quantification absolue est effectuée par rapport à un plasmide de référence contenant les régions amplifiées par PCR quantitative et dont le nombre de copie est connu.

**Tableau A : tampons utilisés au cours du procédé**

| | Tampons | pH | Sucrose (Sigma-Aldrich) | MgCl₂ (Sigma-Aldrich) |
|---|---|---|---|---|
| tampon A | L-Histidine 20mM (Sigma-Aldrich) | 5,0 | 5% W/V | 2mM |
| tampon B | Bis-Tris 20mM (Sigma-Aldrich) | 5,5 | | |
| tampon C | Bis-Tris 20mM (Sigma-Aldrich) | 6 | | |
| tampon D | PBS (GIBCO^{®}) | 7,2 | | |
| tampon E | Bis-Propane 20mM (Sigma-Aldrich) | 8 | | |

### Résultats

### Culture de cellules et clarification:

Ces étapes consistent à produire des vecteurs rétroviraux et lentiviraux en utilisant des cellules stables telles que les PG13 caractérisées par une production stable et en continu des vecteurs rétroviraux en culture continue avec un échange de milieu régulier ou des cellules telles que les HEK293 ou HEK293T qui doivent être transfectées par 3 ou 4 plasmides (apportant les fonctions 'helper' du lentivirus et la séquence du vecteur recombinant) en vue de l'induction de la production des vecteurs lentiviraux. La production transitoire est limitée dans le temps et permet une ou plusieurs récoltes quelques jours après la transfection. Les titres en général dépendent de la construction (séquence) du vecteur mais aussi de la protéine d'enveloppe. Les titres suivants peuvent être obtenus avec ces systèmes de production (tableau 1).

**Tableau 1. Concentrations de vecteurs obtenues avec les différents systèmes de production :**

| **Cellule de production, vecteur-pseudotype** | **Concentration en vecteur (TU/ml, gi/ml)** | **Références** |
|---|---|---|
| PG13, MLV-GaLV-TR | 5^{e}6 TU/ml | Miller et al. 1991 |
| HEK293T, LV (HIV-1) - GaLV-TR | 5^{e}5 TU/ml | Sakuma et al. 2010 |
| HEK293T, LV (HIV-1)-VSVg | 1-5^{e}7 TU/ml | Merten et al. 2011 |

Avant tout traitement suivant il est possible d'éliminer les débris cellulaires et les agrégats présents dans le surnageant de la production. Classiquement on utilise un filtre de 0,45 µm (acétate de cellulose). Le rendement de cette étape est de 80 ± 5%. Cependant l'homme du métier peut utiliser d'autres membranes ou des cascades de membranes, caractérisées par un comportement et rendement similaire.

### Filtration à flux tangentiel:

La filtration à flux tangentiel comprend deux étapes successives d'ultrafiltration et de diafiltration (UF/DF). Ces deux étapes permettent d'éliminer une grande partie des contaminants dont la taille est inferieure à la taille d'exclusion des pores de la membrane utilisée. Cette étape UF/DF permet aussi de concentrer les particules virales et de réduire le volume du produit à purifier. Une membrane de 110cm² avec une taille d'exclusion des pores de 750 kDa (GE HealthCare) a été utilisée. Avant de commencer l'UF, différentes concentrations de sucrose (notamment 5% de sucrose (poids/volume)), et différentes concentrations de MgCl₂ (notamment 2mM MgCl₂ (concentration finale)) sont ajoutées au produit clarifié. Ensuite l'étape de concentration par UF se fait à un flux de 80ml/mn, 7psig, Le réservoir de la TFF est placé dans un bac à glace pour assurer une basse température durant l'UF/DF. L'étape de diafiltration commence après avoir réduit le volume de 500ml à 20ml au cours de l'UF. Pour la DF on utilise 200ml (10 volumes du produit concentré) du tampon de diafiltration : PBS, 5% sucrose, 2mM MgCl₂. A la fin de cette étape on récupère 20ml de produit UF/DF dans un tube Corning 50ml. Le choix du tampon dépend de l'utilisation de la préparation ou des conditions optimales de l'étape suivant la concentration/diafiltration (par ex. : dans ce cas, d'autres tampons peuvent être utilisés comme le Bis-Tris (pH 6,0), 5% sucrose 2mM MgCl₂) - cf. tableau A. Les échantillons sont titrés sur des cellules HCT116 comme décrit par Fenard et al. (2013).

### Etudes d'optimisation des conditions de concentration/diafiltration :

1. Les particules lentivirales ont un diamètre allant de 80 à 120 nm signifiant que la taille de pore de membranes utilisables pour la concentration/diafiltration peut aller au max. jusqu'à 50 nm environ (ou 750 kDa). Dans le cadre de cette invention les tailles de coupure de 500 kDa et de 750 kDa one été évaluées. Les rendements (en TU) ont été les suivants : 64% pour la membrane 750kDa contre 34% de rendement en TU pour la membrane 500kDa.
   La figure 2 présente les gels d'électrophorèse (SDS-PAGE et Western Blot) pour les préparations de vecteurs après filtration tangentielle en utilisant des membranes avec une coupure de 500 kDa et de 750 kDa. En plus des rendements plus élevés obtenus lors de l'utilisation des membranes de 750 kDa, il est clair qu'une coupure de 750 kDa a eu un effet positif (Fig. 2, colonnes 2, 3) par rapport à l'utilisation de la membrane de 500 kDa au niveau de l'élimination des protéines contaminantes (Fig. 2, colonnes 5, 6). De plus, le concentré généré avec la membrane à 750 kDa contient des bandes de protéines beaucoup moins intenses qu'observées pour le surnageant brut.
2. Étant donné que l'étape de filtration tangentielle est caractérisée par la génération de champs de cisaillement conduisant à l'inactivation des particules rétrovirales/lentiviral, il a été nécessaire d'optimiser cette étape en vue de maintenir la fonctionnalité de ces vecteurs. L'ajout d'un polyol à différentes concentrations a été évalué en vue de protéger le vecteur lentiviral des conditions adverses de la filtration tangentielle.

**Tableau 2. Rendement de concentration/diafiltration de LV-GaLV-TR en utilisant différentes concentrations de sucrose.**

| | **Rendement % (TU)** |
|---|---|
| **0% sucrose** | 50,83 |
| **2% sucrose** | 80,31 |
| **5% sucrose** | 80,40 |
| **10% sucrose** | 52,37 |
| **15% sucrose** | 68,49 |
| Note : 190 ml de surnageant brut ont été concentrés à 17 ml et plusieurs fois diafiltrés avec du PBS (pH 7) + différents % de sucrose et 2mM MgCl₂. | |

Ces résultats montrent bien l'intérêt d'effectuer la concentration/diafiltration de surnageant contenant des vecteurs LV-GaLV-TR en présence de sucrose et MgCl₂. Les meilleurs rendements sont obtenus à des concentrations de 2% à 5% en sucrose (Tableau 2).

De plus, l'utilisation d'une concentration modérée en sucrose a l'avantage que l'échantillon à concentrer est moins visqueux car des concentrations élevées en sucrose (10% - 15%) conduisent à une augmentation de la viscosité.
3. Evaluation du pH et de son effet sur la filtration tangentielle et le rendement en vecteur fonctionnel :
Dans la demande FR 13 58909 déposée par la présente demanderesse, il a été montré que la production de vecteurs enveloppés pseudotypés avec différentes protéines d'enveloppe est augmentée lors de l'utilisation d'un pH 6,0 (jusqu'à 2x). Il a été décidé d'évaluer l'impact du choix du pH du surnageant contenant les vecteurs lentiviraux sur l'efficacité de la filtration tangentielle. Dans ce contexte, deux différents pH ont été évalués (pH 6 et pH 7) pendant la concentration/diafiltration des vecteurs lentiviraux pseudotypés GaLV-TR (Tableau 3). La réduction du pH de 7.0 à 6.0 a conduit à une réduction du rendement d'environ 10% (de 73.6% à 64%). Ce rendement reste toutefois acceptable et il est donc possible d'envisager une concentration/diafiltration à pH acide.

**Tableau 3. Impact du pH du surnageant à concentrer / du tampon de diafiltration sur les rendements de concentration/diafiltration des vecteurs lentiviraux pseudotypés GaLV-TR et VSV-g.**

| **Condition de filtration tangentielle** | **Vecteur LV** | **Rendement (%, TU)** |
|---|---|---|
| PBS, 5% sucrose 2mM MgCl₂, pH 7.0 | LV-GaLV-TR | 73,64 |
| BISTRIS 20Mm, 5% sucrose, 2mM MgCl₂, pH 6.0 | LV-GaLV-TR | 63,99 |

4. Identification de la meilleure condition pour la concentration/diafiltration des vecteurs lentiviraux GaLV-TR : En ce qui concerne les lentivirus GaLV-TR, la meilleure condition de concentration/diafiltration (filtration tangentielle) a été la suivante : les vecteurs LV-GaLV-TR (1L) sont clarifiés à travers une membrane 0.45 µm en acétate de cellulose, en présence de 5% sucrose et 2mM MgCl₂, suivi par l'étape de TFF (cartouche 750kDa, 410cm²) avec réduction du volume à atteindre de 20ml (50X). Une étape de diafiltration est ensuite réalisée contre un volume de 200ml de tampon approprié (par exemple : Bis-Tris 20mM pH 6,0, 5% sucrose et 2mM MgCl₂ ou PBS pH 7,0, 5% sucrose et 2mM MgCl₂). Le rendement de cette étape pour les vecteurs LV-GaLV-TR est de 86%±5%, pour un volume de départ de 550ml du produit brut. Le volume du produit concentré est de 15ml avec un facteur de concentration de 36,6X et l'élimination des contaminants atteint plus de 90%.
5. Evaluation des conditions établies de filtration tangentielle pour la concentration/ diafiltration d'autres vecteurs rétroviraux et lentiviraux pseudotypés avec différentes protéines d'enveloppe :
Dans la littérature scientifique différentes protéines d'enveloppe ont été évaluées en vue d'étudier et améliorer le tropisme des vecteurs rétroviraux et lentiviraux. Dans ce contexte, les conditions établies pour la concentration/diafiltration des vecteurs lentiviraux GaLV-TR ont été évaluées pour la concentration/diafiltration de vecteurs rétroviraux et lentiviraux pseudotypés avec différentes protéines d'enveloppe (Tableau 4). Les résultats obtenus avec les vecteurs lentiviraux pseudotypés GaLV-TR sont indiqués comme référence.

**Tableau 4. Concentration/diafiltration des vecteurs rétroviraux pseudotypés avec différentes protéines d'enveloppe :**

| **Condition de filtration tangentielle** | **Vecteur rétroviral** | **Rendement % (TU)** |
|---|---|---|
| BISTRIS, 5% sucrose, 2mM MgCl₂, pH 6,0 | MLV-GaLV (PG13) | 94,2 |
| PBS, 5% sucrose, 2mM MgCl₂, pH 7,0 | LV-GaLV-TR | 73,64 |
| BISTRIS, 5% sucrose, 2mM MgCl₂, pH 6,0 | LV-GaLV-TR | 63,99 |
| Bis-Tris 5% sucrose 2mM MgCl₂ pH 6,0 | LV-MV-CMHII | 61,22 |
| PBS, 5% sucrose, 2mM MgCl₂, pH 7,0 | LV-MV-CMHII | 65,67 |
| PBS 5% sucrose, 2mM MgCl₂, pH 7,0 | LV-VSV-g | 107 |
| BISTRIS, 5% sucrose, 2mM MgCl₂, pH 6,0 | LV-VSV-g | 104 |
| Note : MLV-GaLV : retrovirus murin pseudotypé GaLV ; LV-GaLV-TR : lentivirus pseudotypé GaLV-TR ; LV-MV : lentivirus pseudotypé avec l'env du virus de la rougeole (modifée CMHII) ; LV-VSV-g : lentivirus pseudotypé VSV-g | | |

Les résultats présentés dans le Tableau 4 montrent que tous les vecteurs rétroviraux ou lentiviraux pseudotypés avec différentes protéines d'enveloppe peuvent être concentrés en présence de sucrose et de MgCl₂ à un pH 7,0 conduisant à des rendements allant d'environ 74 % pour LV-GaLV-TR à environ 100% pour le VSVg. En ce qui concerne l'utilisation d'un pH de 6,0 aucune différence n'a été observée pour les vecteurs pseudotypés VSVg.

En ce qui concerne les vecteurs lentiviraux pseudotypés GaLV-TR, ces vecteurs se sont avérés plus stables à pH 7,0 lors de la filtration tangentielle. Le rendement de concentration/diafiltration à été supérieur à 90%, tandis que le rendement a été autour de 74% pour les vecteurs lentiviraux GaLV-TR.

### Chromatographie échangeuse d'anions:

L'étape de concentration/diafiltration par filtration à flux tangentiel a considérablement réduit la charge en protéines et ADN (voir ci-dessus) signifiant qu'une partie importante de contaminants qui pourraient être des compétiteurs des vecteurs à purifier pour l'accès aux ligands de la chromatographie est diminuée. En principe, selon l'utilisation ultérieure on peut imaginer deux manières différentes d'envisager la purification. Elles sont présentées sur la Fig. 1 : un procédé simplifié mettant en oeuvre une seule étape de chromatographie d'exclusion (A sur la fig. 1) et un procédé plus élaboré mettant en oeuvre une étape supplémentaire de chromatographie échangeuse d'anions en vue de la préparation de vecteurs lentiviraux pour utilisation clinique (B en fig. 1).

Les différentes possibilités de chromatographie sont développées par la suite : Juste après l'étape de la TFF UF/DF et afin de diminuer les contaminants et bien séparer les particules virales une étape de chromatographie échangeuse d'anions est ajoutée. Cette technique permet de séparer les biomolécules selon leurs points isoélectriques en fonction du pH et de la concentration en sels. Donc, à une valeur de pH donnée, une certaine concentration en sels (souvent NaCl) est requise en vue de décrocher les biomolécules retenues et cette concentration doit être choisie selon la force d'interaction entre les biomolécules et les ligands : plus cette interaction est élevée plus la concentration en sels (salinité) doit être élevée. De plus, plus le pH du tampon de chromatographie est près du point isoélectrique de l'espèce de biomolécules à purifier, moins de sel est nécessaire pour décrocher les biomolécules des ligands chromatographiques. Cependant il est connu que les vecteurs rétroviraux et lentiviraux perdent rapidement leur pouvoir infectieux en fonction de la concentration en sel (revue par Segura et al. 2006). Donc, dans un premier temps, la stabilité des vecteurs lentiviraux envers différentes concentrations de NaCl a été évaluée.

### 1. Impact de la salinité sur la stabilité des vecteurs lentiviraux GaLV-TR :

Comme indiqué plus haut, l'élution des biomolécules retenues par une colonne de chromatographie se fait très souvent avec des gradients de sel (des tampons contenant du NaCl) ou une étape d'augmentation de la concentration en sel (NaCl). Donc, en vue d'évaluer l'effet de la concentration en NaCl, des tests d'incubation de vecteurs lentiviraux post-TFF ont été réalisés dans différentes concentrations en NaCl allant de 50mM à 1500mM, à température ambiante durant 4h. La fig. 3 représente l'infectivité des vecteurs lentiviraux à température ambiante en fonction de la concentration en NaCl par rapport aux conditions sans NaCl ajouté ou la même préparation de vecteurs incubée à 4°C sans NaCl ajouté. Ce test montre clairement qu'une concentration de NaCl comprise entre 50mM et 1M a un effet modérément néfaste sur la stabilité des vecteurs lentiviraux GaLV-TR avec une perte de l'infectivité allant de 29,52% (50mM NaCl) à 43,86% (1M NaCl) (pourcentage par rapport à la préparation stockée à 4°C). Par contre, la concentration de 1,5M de NaCl conduit à une perte de 63,8% de l'infectivité lorsque la préparation de vecteurs est stockée à température ambiante durant 4h. Il est à noter que le stockage à 20°C (température ambiante) durant 4h sans NaCl ajouté conduit également à une certaine perte de l'infectivité des vecteurs d'environ 23% par rapport au stockage à 4°C.

Ces résultats signifient qu'il est indispensable d'éluer les vecteurs lentiviraux GaLV-TR des supports chromatographiques avec une salinité la moins élevée possible, donc idéalement en dessous de 1M de NaCl en vue de maintenir l'infectivité au maximum. De plus, il est également préférable d'effectuer la totalité de la purification (toutes les étapes) à une température réduite (idéalement entre 4°C et 10°C).

### 2. Evaluation de différents supports de chromatographie échangeuse d'anions (AEX) :

Nous avons utilisé des supports de chromatographie par échange d'anions faible (DEAD (D)) afin de déterminer s'il était possible de limiter l'inactivation des vecteurs avec ce type de support, en particulier en tentant de diminuer la concentration de sel nécessaire pour décrocher lesdits vecteurs de la colonne de chromatographie. Dans des tests préliminaires utilisant un surnageant concentré en provenance de cultures de cellules PG13 (MLV-GaLV) il a été possible de montrer que l'utilisation d'un support chromatographique à base DEAE (Tosoh TSK gel DEAE 5PW) conduit à un rendement en vecteur infectieux de 71% environ rendement plus élevé que lors de l''utilisation d'un échangeur fort (Q Sepharose FF de GE HealthCare) dont le rendement a été de seulement 16%, dû à l'interaction trop forte conduisant à une inactivation lors de l'élution. Dans cet exemple, la concentration de sel nécessaire pour décrocher les vecteurs rétroviraux a été de 655 mM et 915 mM, respectivement.

En se basant sur ces résultats, des échangeurs d'anions faibles ont été choisis pour la suite du développement : plusieurs supports de chromatographie ont été évalués : Monolithe CIM D (DEAE), Poros D50 (Life Technologies), Sartobind D (Sartorius) (Bandeira et al. 2012). Toyopearl 650C DEAE (Merten et al. 2011).

Comme tests préliminaires, trois supports ont été évalués en vue de la purification des vecteurs lentiviraux GaLV-TR à un pH 5,5 ou 6,0 et 7,0. Pour tous les supports testés à un pH 5,5 ou 6,0 et 7,0, le choix du pH faible (5,5 ou 6,0) a été bénéfique au niveau de rendement en vecteur infectieux : en ce qui concerne le support CIM D DEAE le rendement a été augmenté de 23% (pH 7,0) à 64% (pH 6,0) lors de la réduction du pH des tampons utilisés pour la chromatographie de 7,0 à 6,0 (fig. 4). Des résultats similaires ont été observés pour les supports Sartobind 75D (augmentation du rendement de 5,8% à 15,6%) et Poros D (augmentation du rendement de 32% (pH 7,0) à 80.2% (pH 6,0) et environ 100% (pH 5,5)), et pour le gel Toyopearl 650C (augmentation du rendement de 23% (pH 7,0) à 89% (pH 5,5)) (fig. 4). De plus, en vue de décrocher les vecteurs des supports, la salinité du tampon d'élution a pu être plus faible lors de chromatographie à pH 6,0 (donc, plus doux pour les vecteurs lentiviraux). En ce qui concerne le support Poros D utilisé à pH 6,0, l'élution des vecteurs se fait à 650mM NaCl, (voir plus bas). En termes d'efficacité générale, les supports 'modernes' (développés plus récemment, générant des forces de cisaillement réduites (essentiellement dues à la porosité plus importante que pour les autres supports) pendant la chromatographie et caractérisés par l'incompressibilité du support lors de la modification du débit du tampon, comme le monolithe CIM D DEAE ou Poros D) ont montré des rendements supérieurs aux rendements des supports à membrane (Sartobind 75D) ou des supports à base de gel compressible (Toyopearl 650C).

Finalement le choix s'est porté sur les supports récents car leur efficacité de séparation et de recouvrement en vecteurs a été supérieure par rapport aux supports plus classiques. Ces deux supports ont donc été plus largement évalués et leur utilisation a été optimisée en vue de la purification de vecteurs lentiviraux. Les deux supports, CIM D DEAE et Poros D, présentent un rendement intéressant supérieur à 60%. L'élution se fait à 650mM NaCl, Bis-Tris 5% sucrose 2mM MgCl₂ pH 6,0. Une augmentation du pH du tampon d'élution à 7,0 (PBS) entraîne une chute du rendement à une valeur inférieure à 7%, mais l'ajout de sucrose 5% au PBS entraîne une augmentation signification d'environ 7% à 40%. Il est toutefois dans ce cas nécessaire d'utiliser une concentration en NaCl supérieure à 1M. En effet il a été constaté qu'à pH 7,0 pour l'élution des vecteurs (tampon sans sucrose ajouté) il faudra environ 1,5M NaCl dans du PBS, ce qui est probablement l'explication du faible rendement. L'effet négatif de la concentration des sels sur la stabilité des particules virales est connu (Segura et al. 2005).

### 3. Evaluation de différentes valeurs de pH sur l'efficacité de chromatographie en utilisant le support Poros D :

Le pH a été varié dans une fourchette de 5,5 à 8,0 en présence et absence de 5% de sucrose. La présence de sucrose à 5% a un effet positif au niveau du rendement pendant l'étape de la chromatographie échangeuse d'anions lorsque le pH est supérieur à 5,5 (exemple : Poros D) (Tableau 5). L'effet positif de la présence de sucrose sur le rendement n'est plus observé à pH 5,5. Par contre, la présence de sucrose est indispensable à un pH 8,0 en vue de récupérer environ 58% de vecteurs infectieux. Tandis que lors de l'utilisation de pH allant de 6,0 à 7,0, le rendement est entre 52 et 65%, le meilleur rendement (environ 100%) est obtenu à un pH de 5,5.

De manière générale, la présence de sucrose à 5% conduit à une réduction de la salinité nécessaire à l'initiation de l'élution du vecteur lentiviral avec une diminution de la concentration en NaCl nécessaire d'environ 25mM.

**Tableau 5. Comparaison des rendements de LV-GaLV-TR par chromatographie sur Poros D en utilisant des tampons de différents pHs (5,5 - 8,0) en présence ou absence de sucrose.**

| | **Avec/sans sucrose** | **Rendement en TU %** |
|---|---|---|
| pH 5.5 (Bis-Tris) | 5% sucrose | 105,98 |
| | 0%sucrose | 101,33 |
| pH 6.0 (Bis-Tris) | 5% sucrose | 52,32 |
| | 0%sucrose | 29,29 |
| pH 7.0 (PBS) | 5% sucrose | 65,52 |
| | 0%sucrose | 10 |
| pH 8.0 (Bis-Tris-propane) | 5% sucrose | 57,76 |
| | 0%sucrose | 0 |

### 4. Evaluation d'un protocole alternatif comprenant une chromatographie échangeuse d'anions comme première étape :

Nous avons évalué le rendement obtenu lors de l'application d'une étape de chromatographie échangeuse d'anions immédiatement après l'étape de clarification. Dans ces conditions, le rendement observé est plus faible que lorsqu'une étape d'ultrafiltration/diafiltration est mise en oeuvre entre la clarification et la chromatographie échangeuse d'anions. Ce dernier protocole a donc été sélectionné pour la suite de la purification.

### Chromatographie d'exclusion:

La chromatographie d'exclusion est une méthode de choix pour la séparation des biomolécules selon leur taille moléculaire permettant ainsi de séparer les particules des contaminants.

Le gel de filtration Capto Core 700 (GE HealthCare) a été utilisé, mais d'autres supports peuvent être envisagés. Cette étape nous permet de remplacer le tampon de l'étape précédente par le tampon de formulation voulu, d'éliminer les molécules contaminantes de taille inférieure à 750 kDa et d'éviter la dilution de l'échantillon à charger. Cette étape de chromatographie peut être directement utilisée après la filtration à flux tangentiel (concentration/diafiltration - procédé A) ou après une étape de chromatographie par échangeur d'ion - procédé B) (Fig. 1). L'échantillon issu de la filtration à flux tangentiel ou l'échantillon issu des fractions de la chromatographie échangeuse d'anions contenant les vecteurs lentiviraux est chargé sur la colonne de chromatographie d'exclusion. Dans les deux cas le rendement de cette étape est de 86% ±4, selon les fractions retenues pour utilisation ultérieure.

La fig. 5 présente la purification des vecteurs lentiviraux (concentrés et diafiltrés par filtration à flux tangentiel) par filtration sur gel (Capto Core 700). Le pic d'élution du vecteur se trouve au front de passage du tampon et sort de la colonne au niveau des fractions 4-9, recouvrant environ 70% de la quantité des vecteurs initialement chargés sur la colonne (Fig. 5a). La fig. 5B et 5C représentent l'analyse de chaque fraction par électrophorèse (Western Blot, SDS-PAGE) indiquant clairement l'absence des bandes contaminants (fig. 5c) et la présence de la bande à 24-25 kDa correspondant à la protéine p24 de la capside du vecteur lentiviral.

### Rendements et puretés :

Les paramètres les plus importants concernent le rendement global ainsi que la pureté de la préparation de vecteurs lentiviraux au niveau de la réduction de charge en protéines contaminantes et en ADN contaminant.

En ce qui concerne le protocole B (comportant une TFF, une chromatographie échangeuse d'anions (AEX) et une chromatographie d'exclusion (SEC)) (Fig. 1) destiné à la purification des vecteurs lentiviraux à utilisation clinique, le rendement est environ 50% et ce protocole permet l'élimination 99,9% de protéines contaminants et 99,9 %, d'ADN contaminant.

Le protocole A (comportant une TFF et une chromatographie d'exclusion (SEC)) (Fig. 1) destiné à la purification des vecteurs lentiviraux à utilisation en recherche est plus simple, car dépourvu de l'étape de chromatographie échangeuse d'ions. Le rendement global est plus élevé en raison de la réduction du nombre d'étapes de purification et atteint 60,2%, l'élimination des contaminants ADN résiduel de ce protocole simplifié est de l'ordre de 96,17% et on observe une réduction des protéines contaminants de 99,63%.

### Exemples pratiques de transduction de cellules cibles :

### Transduction des cellules CD34+ :

Pour déterminer la qualité des vecteurs purifiés, des cellules CD34+ de sang de cordon sont transduites. Les cellules sont décongelées, après 18 heures de pré-stimulation par des cytokines. La transduction se fait pendant 6 heures. Ensuite, les cellules sont mises dans un milieu de différenciation pendant 5 jours. Les cellules sont ensuite passées au FACS FC500 (BD Biosciences) pour mesurer le pourcentage d'expression de la GFP. Les résultats suivants sont typiquement obtenus (fig. 6) : la purification par concentration/diafiltration des vecteurs lentiviraux (GaLV-TR) conduit à une augmentation de l'efficacité de transduction des cellules CD34+ (exprimée en pourcentage de cellules exprimant la GFP) allant de 9% lors de l'utilisation d'un surnageant brute à 70% pour l'utilisation d'une préparation de vecteurs LV concentré/diafiltré.

### Purification d'un vecteur lentiviral pseudotypé au moyen d'une enveloppe modifiée du virus de la rougeole

On décrit ici un procédé de purification d'un vecteur lentiviral pseudotypé au moyen de la glycoprotéine modifiée d'enveloppe du virus de la rougeole (pseudotypage MV). Les vecteurs lentiviraux LV-MV-CMHII (CMHII = anti-CMHII) produits selon la procédure indiquée ci-dessus sont purifiés selon les étapes suivantes:
1) concentration/diafiltration au moyen d'une étape de TFF
   - membrane utilisée: GE #UFP-750-E-3MA 110cm², pour la purification d'un litre de produit
   - tampon de diafiltration: PBS ((pH 7,0), 2mM MgCl₂, 5% sucrose)
   - reduction de volume: de 500ml/1000ml à 20ml, le tampon étant remplacé par le tampon de diafiltration
   - rendement en vecteurs infectieux: 64-70%
2) chromatographie d'exclusion (gel filtration):
   - colonne utilisé : CaptoCore 700 4.7ml
   - tampon de formulation: PBS, 5% sucrose, 2mm MgCl₂, ou bien X-vivo ou HANKS, contenant 5% sucrose et 2mM MgCl₂
   - équilibrage de la colonne avec 10 CV d'un tampon de formulation
   - chargement du concentré de la TFF sur la colonne CaptoCore700 à une vitesse de 0.5mL/min
   - lavage de la colonne avec 20 CV du tampon de formulation
   - Récolte des échantillons correspondant au pic de DO (volume 21 mL à 50X)
   - rendement en vecteurs infectieux: >90% en TU

Le rendement global de cette purification est de 60 à 63% en vecteurs infectieux, ce qui représente une avancée majeure pour la purification, et donc l'exploitation, de vecteurs lentiviraux pseudotypés au moyen de la glycoprotéine MV modifiée.

**REFERENCES BIBLIOGRAPHIQUES**

## Revendications

1. Procédé pour la purification d'un lentivirus pseudotypé avec la glycoprotéine d'enveloppe GaLV-TR, VSV-g ou MV, comprenant une étape de chromatographie échangeuse d'anions, les tampons utilisés au cours de ladite chromatographie étant de pH compris entre 5 et 5,9 ; dans lequel la chromatographie échangeuse d'anions est une chromatographie échangeuse d'anions faible.

2. Procédé selon la revendication 1, le pH des tampons étant égal à 5,5.

3. Procédé selon l'une quelconque des revendications précédentes, l'étape de chromatographie échangeuse d'anions étant précédée d'une étape d'ultrafiltration/diafiltration, en particulier une filtration à flux tangentiel.

4. Procédé selon la revendication 3, l'étape d'ultrafiltration/diafiltration comprenant l'utilisation d'un ou plusieurs tampons de pH compris entre 5,5 et 7,5 comprenant éventuellement un polyol.

5. Procédé selon l'une quelconque des revendications précédentes, pour la purification d'un lentivirus, à partir du milieu de culture d'une culture cellulaire de cellules productrices dudit lentivirus, le procédé comprenant:
(a) la clarification du milieu de culture cellulaire, notamment par filtration du milieu de culture sur un filtre de rétention dont le seuil de rétention est compris entre 0,2 et 0,45 µm;
(b) une étape d'ultrafiltration/diafiltration des virus clarifiés, notamment au moyen d'une filtration à flux tangentiel;
(c) une chromatographie échangeuse d'anions;
(d) une chromatographie d'exclusion ; notamment une résine d'exclusion présentant une taille d'exclusion comprise entre 300 et 1000 kDa.

6. Procédé selon la revendication 5, la résine utilisée pour la chromatographie d'exclusion étant multimodale, présentant une double fonctionnalité d'exclusion et d'adsorption.

7. Procédé selon l'une quelconque des revendications 4 à 6, le polyol étant choisi parmi le sucrose, le mannitol, le sorbitol et le tréhalose.

8. Procédé selon l'une quelconque des revendications 1 à 7, les virus purifiés étant produits en milieu neutre ou en milieu modérément acide, en particulier à un pH compris entre pH 6 et pH 7.

9. Procédé selon l'une quelconque des revendications 4 à 8, le polyol étant présent dans le tampon à une concentration comprise entre 1,5 % et 15 % en poids dans le tampon, en particulier entre 2 % et 5 %, plus particulièrement à 5 %.

10. Procédé selon l'une quelconque des revendications précédentes, les tampons utilisés au cours dudit procédé comprenant également un sel de magnésium, notamment du chlorure de magnésium, notamment à une concentration comprise entre 0,1 mM et 5 mM, en particulier entre 1 et 3 mM, plus particulièrement à 2 mM.

11. Procédé selon l'une quelconque des revendications précédentes, dans lequel la chromatographie échangeuse d'anions est une chromatographie échangeuse d'anions sur colonne.

## Patentansprüche

1. Ein Verfahren zur Reinigung eines Retrovirus, insbesondere eines Lentivirus, gegebenenfalls mit dem Hüllglycoprotein GaLV, GaLV-TR, VSV-g oder MV pseudotypisiert, umfassend einen Schritt der Anionenaustausch-Chromatographie, wobei die im Verlauf besagter Chromatographie verwendeten Puffer sind
- pH-Puffer zwischen 5 und 5,9;
wobei es sich bei der Anionenaustausch-Chromatographie um eine schwache Anionenaustausch-Chromatographie handelt.

2. Verfahren gemäß Anspruch 1, wobei der pH der Puffer gleich 5,5 ist.

3. Verfahren gemäß einem der vorhergehenden Ansprüche, wobei dem Schritt der Anionenaustausch-Chromatographie ein Ultrafiltrations-/Diafiltrationsschritt, insbesondere eine Tangentialflussfiltration, vorausgeht.

4. Verfahren gemäß Anspruch 3, wobei der Ultrafiltrations-/Diafiltrationsschritt die Verwendung eines oder mehrerer pH-Puffer zwischen 5,5 und 7,5, umfassend gegebenenfalls ein Polyol, umfasst.

5. Verfahren gemäß einem der vorhergehenden Ansprüche, zur Reinigung eines Lentivirus aus dem Kulturmedium einer Zellkultur von produzierenden Zellen des besagten Lentivirus, wobei das Verfahren umfasst:
(a) Klärung des Zellkulturmediums, insbesondere durch Filtration des Kulturmediums auf einem Retentionsfilter, dessen Retentionsschwelle zwischen 0,2 und 0,45 µm beträgt;
(b) einen Ultrafiltrations-/Diafiltrationsschritt der geklärten Viren, insbesondere mithilfe einer Tangentialflussfiltration,
(c) eine Anionenaustausch-Chromatographie,
(d) eine Ausschlusschromatographie; insbesondere ein Ausschlussharz mit einer Ausschlussgröße zwischen 300 und 1000 kDa.

6. Verfahren gemäß Anspruch 5, wobei das für die Ausschlusschromatographie eingesetzte Harz multimodal ist, das eine doppelte Funktionalität von Ausschluss und Adsorption aufweist.

7. Verfahren gemäß einem der Ansprüche 4-6, wobei das Polyol aus Sucrose, Mannit, Sorbit und Trehalose ausgewählt ist.

8. Verfahren gemäß einem der Ansprüche 1 bis 7, wobei die gereinigten Viren in einem neutralen Medium oder einem leicht sauren Medium, insbesondere bei einem pH zwischen 6 und 7, produziert werden.

9. Verfahren gemäß einem der Ansprüche4 bis 8, wobei das Polyol im Puffer mit einer Konzentration zwischen 1,5 und 15 Gew.-%, insbesondere zwischen 2 und 5 Gew.-%, ganz besonders mit 5 Gew.-% vorliegt.

10. Verfahren gemäß einem der vorhergehenden Ansprüche, wobei die im Verlauf besagten Verfahrens verwendeten Puffer ebenfalls ein Magnesiumsalz, insbesondere Magnesiumchlorid, insbesondere mit einer Konzentration zwischen 0,1 mM und 5 mM. insbesondere zwischen 1 und 3 mM, ganz besonders mit 2 mM, umfassen.

11. Verfahren gemäß einem der vorhergehenden Ansprüche, wobei die Anionenaustausch-Chromatographie eine Anionenaustausch-Säulenchromatographie ist.

## Claims

1. Process for purifying a lentivirus, pseudotyped with the envelope glycoprotein, GaLV-TR, VSV-g or MV, comprising an anion-exchange chromatography step, the buffers used during said chromatography having a PH between 5 and 5.9, wherein the anion-exchange chromatography is a weak-anion exchange chromatography.

2. Process according to claim 1, the pH of the buffers being equal to 5.5.

3. Process according to any one of the preceding claims, the anion-exchange chromatography step being preceded by an ultrafiltration/diafiltration step, in particular tangential flow filtration.

4. Process according to claim 3, the ultrafiltration/diafiltration step comprising the use of one or more buffers having a pH between 5.5 and 7.5 optionally comprising a polyol.

5. Process according to any one of the preceding claims, for purifying a lentivirus from the culture medium of a cell culture of cells producing said lentivirus, the process comprising:
(a) clarification of the cell culture medium, in particular by filtering the culture medium through a retentive filter for which the retention threshold is between 0.2 and 0.45 µm;
(b) an ultrafiltration/diafiltration step for the clarified viruses, in particular by means of tangential flow filtration;
(c) an anion-exchange chromatography;
(d) an exclusion chromatography; in particular an exclusion resin having an exclusion size between 300 and 1000 kDa.

6. Process according to claim 5, the resin used for the exclusion chromatography being multimodal, having a dual functionality of exclusion and adsorption.

7. Process according to any one of claims 4 to 6, the polyol being selected from sucrose, mannitol, sorbitol and trehalose.

8. Process according to any one of claims 1 to 7, the purified viruses being produced in a neutral medium or in a moderately acidic medium, in particular at a pH between pH 6 and pH 7.

9. Process according to any one of claims 4 to 8, the polyol being present in the buffer at a concentration between 1.5% and 15% by weight in the buffer, in particular between 2% and 5%, more particularly at 5%.

10. Process according to any one of the preceding claims, the buffers used during said process also comprising a magnesium salt, in particular magnesium chloride, in particular at a concentration between 0.1 mM and 5 mM, in particular between 1 and 3 mM, more particularly at 2 mM.

11. Process according to any one of the preceding claims, wherein the anion-exchange chromatography is anion-an exchange column chromatography.
